# EUROPEAN PATENT APPLICATION

(11) **EP 2 374 460 A1**
(43) Date of publication of application: **12.10.2011**
(21) Application number: 08878690.0
(22) Date of filing: 12.12.2008
(51) Int. Cl.: A61K 31/519, A61K 9/54, A61P 15/10

(54) **PULSED-RELEASE SILDENAFIL COMPOSITION AND METHOD FOR PREPARING SAID COMPOSITION**

(71) Applicant: Siegfried Rhein S.A. De C.V., 05120 Ciudad de Mexico (MX)
(72) Inventor: FIORE, Esteban Alejandro, 1417 Buenos Aires (AR)
(74) Representative: Eder, Michael
(86) International application number: PCT/IB2008/003466
(87) International publication number: WO 2010/067140

(57) **Abstract**

The present invention relates to a pulsed-release sildenafil pharmaceutical composition comprising an immediate release fraction containing from 5 to 100 mg of sildenafil and a controlled release fraction containing from 25 to 150 mg of sildenafil, wherein the controlled release fraction consists of particles containing (a) a superdisintegrant agent (b) a coating comprising at least one pH-dependent solubility polymer and at least one pH-independent solubility polymer and (c) optionally, other pharmaceutical excipients. The composition of the present invention exhibits a faster dissolution profile in alkaline media than in acid media, which allows for obtaining enhanced pulsed-release formulations.

## Description

### FIELD OF THE INVENTION

The present invention relates to pulsed-release compositions of sildenafil and to the process for preparing the same. Particularly, the present invention relates to pulsed-release sildenafil capsules comprising an immediate release fraction containing from 5 to 100 mg sildenafil and a controlled release fraction containing from 25 to 150 mg sildenafil, wherein said controlled release fraction consists of particulates containing (a) a superdisintegrant agent, (b) a coating formed of at least one pH-dependent solubility polymer and at least one pH-independent solubility polymer, and (c) optionally other pharmaceutical excipients. The composition of the instant invention exhibits a faster dissolution profile in alkaline media than in acidic media, which allows for obtaining enhanced pulsed-release formulations.

### BACKGROUND OF THE INVENTION

Sildenafil is a selective phosphodiesterase type-5 inhibitor, useful in treating erectile dysfunction and pulmonary hypertension. The importance of this enzyme in regulating the physiologic response to sexual stimulation is widely known.

The sildenafil compound was described, as a product, in European Patent EP 463 756 B1, later, European Patent EP 702 55 B1 made reference to its use in treating impotency. Later on, process patents regarding sildenafil preparation were filed; among these are EP 812 845 B1 and US 6 204 383. The latter, issued to Torcan, allows for the use of a unique process for manufacturing the active ingredient.

Then, within the previous art, Patent EP 941 075 B1 claims fast disintegration compositions, which brings immediate action of said active ingredient. Finally, Patent EP 1 123 088 B1 discloses prolonged release of sildenafil in hydrophilic polymers matrices and prolonged release multiparticulate sildenafil compositions, in which the active ingredient is in granulated form coted with insoluble polymers like Eudragit RL (ammonium methacrylate copolymer type A) and Eudragit RS (ammonium methacrylate copolymer type B). As a result, these compositions, as further described below, have low bioavailability.

A major limitation in the use of sildenafil to treat impotency is the compound's relatively short plasmatic elimination half life. Since the sildenafil's plasmatic elimination half-life is of about 4 hours, thus the duration of the therapeutic effect is limited. For this reason, the object of the present invention is to create pulsed-release sildenafil compositions providing a long lasting-effect by means of release pulses and accordingly, allows for the patient to freely take this medication at any time that he considers convenient to himself, without the need to coordinate the administering time with the estimated time at which the therapeutic effect is required an/or to repeat the sexual act. Similarly, the long-lasting therapeutic effect enables a more comfortable and effective use in patients with pulmonary hypertension.

For the purpose of the present invention, it is understood that an immediate release sildenafil composition is one composition that releases 80% or more of its contents within 30 minutes as measured in a USP dissolution apparatus in a media comprising 900 ml of 0.01 M hydrogen chloride solution. Long-lasting release sildenafil compositions are those that release its contents in a time period of greater than 30 minutes, and can release the active ingredient either gradually or in a pulsed fashion (known as pulsed-release), for example in a immediate release pulse an in one or more controlled release pulses.

It is worth mentioning that the sildenafil solubility is adequate in acidic media, like in the stomach, however it is noted that in alkaline media, like that found in the intestine, the solubility of sildenafil decreases markedly. Because sildenafil has a pH-dependent solubility, compositions formed by matrix pills or granules covered by insoluble membranes, as those exemplified in the previous art, give dissolution profiles in alkaline media that are much slower than those obtained in acidic media. In the referenced patent EP 1 123 088 B1 the dissolution profile of prolonged release sildenafil compositions is measured in three different media (pH 2, pH 4.5 and pH 7.5) and obtaining seemingly equivalent dissolution profiles, however the direct comparison of the curves thereof is not right, because the dissolution was obtained by using, in each case, 1 liter in volume for both pH 2 and pH 4.5, and 5 liters in the case of pH 7.5. This kind of compositions shows reduced intestinal release issues and consequently low bioavailability. On the contrary, the composition of the present invention shows a faster dissolution profile in alkaline media than an acidic media.

By these reasons, it was proposed to find a composition, which meets these requirements and overcome the above disadvantages, thus leading to the present invention. The results obtained have been very beneficial since a pulsed-release sildenafil composition has been attained providing for an appropriate in-vivo dissolution level, particularly in alkaline media like that found in the intestine. After several essays, it was found that the composition of the present invention overcomes the low solubility issues in alkaline media, by using particles containing sildenafil along with a superdisintegrant agent, coated with a membrane comprising pH-independent solubility polymers and pH-dependent solubility polymers combined.

Therefore, the object of the present invention refers to compositions having a pulsed-release sildenafil composition and to the process for the preparation thereof. In particular, the present invention refers to pulsed-release sildenafil capsules comprising an immediate release fraction containing from 5 to 100 mg sildenafil and a controlled release fraction containing from 25 to 150 mg sildenafil, wherein said controlled release fraction consists of particulates containing (a) a superdisintegrant agent, (b) a coating formed of at least a pH-dependent solubility polymer and at least a pH-independent solubility polymer, and (c) optionally, other pharmaceutical excipients. The pulsed-release sildenafil compositions of the present invention exhibits a faster dissolution profile of between 40 and 60% of the active ingredient in 30 minutes, between 60 and 80% of the active ingredient in 3 hours, and not less than 80% in 5 hours when measured on a USP apparatus 1 (baskets) at 100 rpm in a media comprising 900 ml of 0.01 M hydrogen chloride solution, during the first two hours and for the remaining time in pH 6.0 media comprising 0.5% sodium chloride with 1% sodium lauryl sulfate.

### BRIEF DESCRIPTION OF THE FIGURES

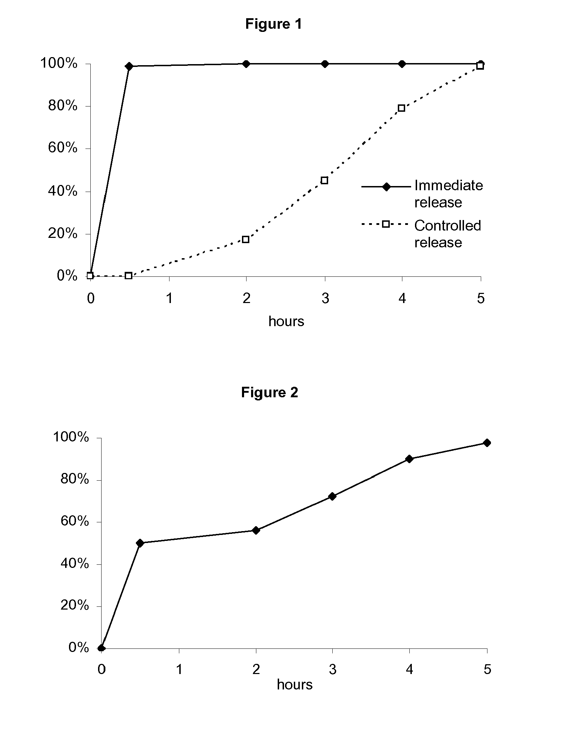

Figure 1 shows the in-vitro dissolution profile of both immediate release and controlled release fractions of Example 1.
Figure 2 shows the in-vitro dissolution profile of Example 1 composition (pulsed-release capsules).

### DETAILED DESCRIPTION OF THE INVENTION

Compositions of the present invention are prepared according to the following process: 1) forming the immediate release fraction; 2) forming the controlled release fraction; and 3) filling capsules with the amount required of each fraction.

### Immediate Release Fraction

The immediate release fraction comprises sildenafil-containing particulates; it can be in the form of granules, microtablets or powder for filling into capsules. In the case of microtablets, these can be made by conventional methods known in the art. Preferably these microtablets are 2.5 mm or less in diameter and have a smooth surface so that they can be coated. Also, in the case of using granules, these are preferred in a spherical shape so that they can be coated. Methods of making spherical immediate release granules are the extrusion-spheronization method and the method of depositing an active ingredient over an inert core. The extrusion-spheronization method requires the use of deformable excipients, like microcrystalline cellulose, which accounts for a high percentage of the composition, thus preventing to obtain formulations with high sildenafil concentration, therefore, using the method of active ingredient deposition over an inert core is preferred.

The general method of depositing sildenafil over inert cores involves the following steps: adding ethanol, purified water, or a mixture of ethanol and purified water into a reactor of suitable capacity. Adding a binder agent and maintaining stirring until complete dissolution of the same. Subsequently, adding sildenafil and, optionally, a superdisintegrant agent, and maintaining stirring until a homogenous dispersion is achieved. Spraying the slurry thus obtained over the inert cores using a fluid bed fitted with a Wurster insert or tangential rotor. Drying the granules obtained in this manner in either a fixed bed or fluid beddryer. Optionally, diluent agents and/or pH buffers can be added to the composition.

### Controlled Release Fraction

The controlled release fraction is comprised of coated particles containing sildenafil and these can be in the form of coated granules or coated microtablets. Preparation of controlled release sildenafil particles involves the following steps:
(a) forming granules containing sildenafil and a superdisintegrant agent by the method of active ingredient deposition over an inert core; alternatively, it is also possible to make microtablets containing sildenafil and a superdisintegrant agent.
(b) preparing a coating solution or slurry consisting of at least one pH-independent solubility polymer and at least one pH-dependent solubility polymer.
(c) spraying the coating solution or slurry over said granules or microtablets; and
(d) drying said coating.

The step of preparing the controlled release fraction by using the method of active ingredient deposition over an inert core consists of: a) adding ethanol, purified water or a mixture of ethanol and purified water into a reactor of suitable capacity; b) adding a binder agent and maintaining stirring until complete dissolution of the same; c) adding sildenafil and the superdisintegrant agent and maintaining stirring until a homogenous dispersion is obtained; d) spraying the slurry thus obtained over inert cores using a fluid bed fitted with a Wurster insert or tangential rotor; f) drying the granules obtained in this manner in either a fixed bed or fluid bed dryer; f) preparing a coating solution or slurry consisting of at least one pH-independent solubility polymer and at least one pH-dependent solubility polymer, either dissolved or dispersed in a suitable solvent; g) spraying the obtained coating solution or slurry over said granules using a Wurster or tangential rotor type fluid bed; and (h) drying said coated granules until a constant weight is achieved.

On the other hand, the step of preparing the controlled release fraction by using the microtablet compression method consists of: a) mixing sildenafil with a diluent and a superdisintegrant agent in a suitable mixer b) granulating the mixture by using a binder agent solution; c) drying and sieving the obtained granulate; d) mixing with lubricant and compressing using microtablet punches; e) preparing a coating solution or slurry comprising at least one pH-independent solubility polymer and at least one pH-dependent solubility polymer, either dissolved or dispersed in a suitable solvent; f) spraying the coating solution or slurry thus obtained over said microtablets using a Wurster or tangential rotor type fluid bed; and g) drying said coated microtablets until a constant weight is achieved.

Once the granules o microtablets containing sildenafil and a superdisintegrant agent are made, a coating solution or slurry comprising at least one pH-independent solubility polymer and at least one pH-dependent solubility polymer is prepared. For this purpose, a reactor is used wherein the polymer mixture is either dissolved or dispersed in a suitable solvent, for example ethanol, isopropyl alcohol, water or a mixture thereof. Then, optionally, a plastifier agent can be added. Once the coating solution or slurry is finished, it is sprayed over said sildenafil granules or microtablets using a Wurster or tangential rotor type fluid bed. Generally, the coating requires drying for several hours until a constant weight is achieved. If desired, a curing step can be applied to the long-lasting release membrane.

Optionally, said granules and microtablets can contain one or more pharmaceutical excipients acting as a binder, a diluent, an inert core, a pH buffer or lubricant.

### Capsule Filling

Once the desired weight of each (immediate release and controlled release) fraction is defined for filling into capsules, it is possible to make a mixture of said fractions and fill the capsules with said mixture. In order to avoid dissociating the mixture, it is preferred to perform the encapsulation in a capsule filling equipment comprising two stations and metering the desired weight of each fraction independently. Generally, the fraction ratio is 40:60 to 60:40 and preferably, is a 50:50 ratio. The capsules can be hard capsules, made of gelatin, hypromellose, pullulan, or other similar polymers, preferably gelatin hard capsules.

Ideally, a sildenafil pulsed release composition should be small enough and of easy administration. To this purpose, it is required for the active ingredient to be highly concentrated, for example to levels higher than 45%, o even better, higher than 60% by weight of the composition. The compositions of the present invention allow for sildenafil concentrations higher than 60% by weight of the composition.

Representative examples of superdisintegrant agents are croscarmellose sodium, sodium starch glycolate and crospovidone, the use of micronized crospovidone being preferred. Generally, the superdisintegrant varies from 1 to 10% and preferable it is between 3 and 5% by weight of the composition.

The binder agent is a soluble polymer selected among hydroxypropylcellulose (HPC), hypromellose (HPMC), povidone (PVP), polyvinyl alcohol (PVA) and polyethylene oxide (PEO). Preferably, low viscosity HPC is used. Binder concentration is between 0.1 and 5% by weight of the composition.

Representative examples of (pH-independent) water-insoluble coating polymers are ethyl cellulose, polyvinyl acetate, neutral ethyl acrylate and methyl acrylate-based copolymers, ammonium methacrylate copolymers and the like. Representative examples of pH-dependent solubility polymers include: cellulose acetate phthalate, hydroxypropylmethyl cellulose phthalate, hydroxypropylmethyl cellulose acetate succinate, polyvinyl acetate phthalate, methacrylic acid copolymers, shellac and the like. Preferably, a mixture of ammonium methacrylate copolymer type B (Eudragit RS100) and methacrylic acid copolymer type C (Eudragit L100-55) is employed. The pH-dependent and pH-independent polymer ratio can vary between 80:20 and 20:80, being preferably 60:40. The coating polymer weight is typically between 2 and 10% by weight of the total composition.

The inert core is preferably spherical in shape. It may comprise sugar, starch mannitol, xylitol, microcrystalline cellulose and organic acids, like fumaric acid, tartaric acid, succinic acid and citric acid, among others. The inert core weight can vary between 1 and 30%. Preferably inert sugar and starch cores are preferred, constituting between 15 and 20% by weight of the composition.

Plasticizers examples include: triacetin, tributyl citrate, triethyl citrate, diethyl phthalate, castor oil, dibutyl sebacate, acetylated monoglycerides, medium chain triglyceride and similar compounds, among these the use of medium chain triglyceride is preferred. Generally, the plasticizer accounts for between 10 and 20% by weight of the coating polymers.

Optionally, the granules can contain one or more excipients, which function both as diluents and pH buffers. Representative examples of said diluents are: lactose, starch, microcrystalline cellulose, methylcellulose, povidone, calcium phosphate, mannitol, sorbitol and gelatin, among others. Representative examples of pH buffers are: organic acids, for example fumaric acid, tartaric acid, succinic acid, citric acid and aspartic acid.

The composition dissolution profile is measured by the following procedure: USP Apparatus 1 (Baskets) at 100 rpm in 900 ml of a 0.01 M hydrogen chloride solution for the first two hours and, for the remaining time, in a pH 6.0 aqueous solution comprising 1% sodium lauryl sulfate and 0.5% sodium chloride (pH is adjusted using a 0.01 M hydrogen chloride solution, if required). Capsules of the present invention release, as measured by a procedure disclosed hereinabove, between 40 and 60% of the active ingredient in 30 minutes, between 60 and 80% of the active ingredient in 3 hours, and not less than 80% in 5 hours.

As shown in the examples of preparation included below, the pulsed-release compositions of present invention were tested for dissolution and compared with compositions described in the previous art. Dissolution was determined on two systems, one without any change in pH and one with a change in pH. The first system (without pH change) was performed in 900 ml of a 0.01 M hydrogen chloride solution in a dissolution test USP apparatus 1 (baskets) at 100 rpm. The second dissolution system (with pH change) was measured on the same equipment using, for the first two hours, a medium comprising a 0.01 M hydrogen chloride solution and a pH 6.0 aqueous solution comprising 1% sodium lauryl sulfate and 0.5 sodium chloride (pH was adjusted using a 0.01 M hydrogen chloride solution, if necessary) for the remaining time.

It was demonstrated that, by using the present invention pulsed-release composition, a faster dissolution profile was obtained in alkaline media than in acidic media.

### PREPARATION EXAMPLES

### Example 1

Capsules comprising a mixture of immediate release granules and controlled release granules, containing a superdisintegrant agent and a coating formed by at least one pH-dependent solubility polymer (Eudragit L100-55) and at least one pH-independent solubility polymer (Eudragit RS100).

**Table 1 - Content per Capsule**

| Ingredient | Function | mg/dose | % |
|---|---|---|---|
| Sildenafil Citrate | Active Ingredient | 140.48 | 65.6% |
| Sugar spheres/starch (500- 600 microns) | Inert Core | 41.52 | 19.4% |
| Plasdone 29-32 (Povidone K30) | Binder | 15.00 | 7.0% |
| Kolidon CL (Crospovidone) | Superdisintegrant | 8.20 | 3.8% |
| Eudragit L100-55 (Methacrylic acid copolymer type C) | pH-Dependent Membrane | 4.80 | 2.2% |
| Eudragit RS100 (Ammonium Methacrylate type B) | pH-Independent Membrane | 3.20 | 1.5% |
| Miglyol 812 N (Medium chain triglycerides) | Plasticizer | 0.80 | 0.4% |
| Total | | 214.00 | 100.0% |

Conclusion: The sildenafil citrate content is higher than 65% by weight of the composition.

### Composition Preparation

First, the slurry containing sildenafil citrate was prepared as follows: a mixture of ethanol and water was placed, in equal portions, in a reactor of suitable capacity. Povidone K30 was added and maintained under stirring until complete dissolution thereof. Then, the sildenafil citrate and micronized crospovidone was added and maintained under stirring until a homogeneous dispersion was obtained. The resulting slurry was sprayed over sugar and starch spheres in a fluid bed fitted with a Vector brand tangential rotor Model FL-M-1 fitted with a 9 inches tangential rotor and under the following operating conditions: air temperature, 29 °C; air flow rate, 22 cfm; rotor speed, 330 ppm; spraying pressure, 13 psi; and peristaltic pump speed, 19 rpm. The granules thus obtained were dried in a fixed bed dryer at a temperature of 40 °C for 20 hours. The prepared granules were partitioned in to equal size portions, one half for the immediate release fraction and the other half for the controlled release fraction. For the controlled release coating, a coating solution comprising a mixture of ethanol Eudragit L100-55 and Eudragit RS100 at a solid ratio of 60:40 and plasticized with Miglyol 812N was prepared and then applied over the immediate release granules using a Vector brand fluid bed fitted with a 6 inches Wurster insert under the following operating conditions: input air temperature, 40 °C; air flow rate, 100 cfm; spraying pressure, 15 psi; and peristaltic pump speed, 10 rpm. The granules were dried in a fixed bed dryer at 40 °C for 5 hours.

Encapsulation was carried out on an automatic capsule filling equipment, MG2 brand, equipped with two stations. Hard gelatin capsules size 2 were filled with immediate release granules containing an equivalent amount of sildenafil of 50 mg and controlled release granules containing an equivalent amount of base sildenafil of 50 mg, accounting for a total filling weight of 214 mg per capsule with a 140 mg sildenafil citrate content.

**Table 2 - Immediate Release Fraction Dissolution from Example 1**

| | System 1 (acidic media) |
|---|---|
| 30 min | 99% |

Conclusion: The immediate release fraction releases all the sildenafil content in 30 minutes.

**Table 3 - Controlled Release Fraction Dissolution from Example 1**

| | System 2 (pH change) |
|---|---|
| 30 min | 0% |
| 3 h | 45% |
| 5 h | 99% |

Conclusion: The controlled release fraction releases sildenafil gradually.

**Table 4 - Dissolution of Pulsed-release Capsules from Example 1**

| | System 1 (acidic media) | System 2 (pH change) |
|---|---|---|
| 30 min | 51% | 50% |
| 3 h | 67% | 72% |
| 5 h | 85% | 98% |

Conclusion: In both media, the composition achieves a complete dissolution (over 80%) after 5 hours. The dissolved percentage in system 2 (media change) is higher than the dissolved percentage by the composition in system 1 (acidic pH).

### Comparative Example A (composition according to the prior art)

Capsules containing a mixture of immediate release granules and controlled release granules, without superdisintegrant agent and coated with an insoluble polymer mixture (Eudragit L100 and Eudragit RS100).

**Table 5 - Content per Capsule**

| Ingredient | Function | mg/dose | % |
|---|---|---|---|
| Sildenafil Citrate | Active Ingredient | 140.5 | 63.00% |
| Granular Succinic Acid (500-600 microns) | Inert Core/pH buffer | 59.6 | 26.73% |
| Plasdone 29-32 (Povidone K30) | Binder | 15.6 | 7.00% |
| Eudragit RL100 (Methacrylic acid copolymer type A) | pH-Independent Membrane | 3.3 | 1.48% |
| Eudragit RS100 (Ammonium Methacrylate type B) | pH-Independent Membrane | 3.3 | 1.48% |
| Miglyol 812 N (Medium chain triglycerides) | Plasticizer | 0.7 | 0.31% |
| Total | | 223.0 | 100.00% |

### Composition Preparation

The same manufacturing method as used in the immediate release granules of Example 1 was employed except for the use of a superdisintegrant. The controlled release granules were coated with a mixture comprising equal portions of Eudragit RL100 and Eudragit RS100 plasticized with triethyl citrate.

**Table 6 - Dissolution of Pulsed-release Capsules fromcomparative Example A**

| | System 1 | System 2 |
|---|---|---|
| | (acidic media without pH change) | (pH change) |
| 30 min | 50% | 52% |
| 3 h | 68% | 63% |
| 5 h | 90% | 75% |

Conclusion: The comparative example composition does not achieve a complete dissolution after 5 hours in system 2 (pH change).

### Example 2

Capsules comprising a mixture of immediate release granules and controlled release granules, containing a superdisintegrant agent and a coating comprising at least one pH-dependent solubility polymer (HP-55) and at least one pH-independent solubility polymer (Ethocel).

**Table 7 - Content per Capsule**

| Ingredient | Function | mg/dose | % |
|---|---|---|---|
| Sildenafil Citrate | Active Ingredient | 140.5 | 66.00% |
| Sugar spheres/starch (500-600 microns) | Inert Core | 41.5 | 19.5% |
| Plasdone 29-32 (Povidone K30) | Binder | 15.1 | 7.1% |
| Kollidon CL (Crospovidone) | Superdisintegrant | 8.2 | 3.8% |
| HP-55 (Hydroxypropylmethyl cellulose phthalate) | pH-Dependent Membrane | 4.2 | 2.0% |
| Ethocel 20 (ethyl cellulose N20) | pH-Independent Membrane | 2.8 | 1.3% |
| Miglyol 812 N (Medium chain triglycerides) | Plasticizer | 0.7 | 0.3% |
| Total | | 213.0 | 100.0% |

Conclusion: The sildenafil citrate content is higher than 65% by weight of the composition.

### Composition Preparation

The same manufacturing method as used in the immediate release granules of Example 1 was employed. The controlled release granules were coated with a mixture comprising Ethyl cellulose N20 and Hydroxypropylmethyl cellulose phthalate in a 40:60 ratio. Ethanol was used as solvent to dissolve polymers.

**Table 8 - Dissolution of Capsules from Example 2**

| | System 1 | System 2 |
|---|---|---|
| | (acidic media without pH change) | (pH change) |
| 30 min | 52% | 51% |
| 3 h | 65% | 75% |
| 5 h | 86% | 96% |

Conclusion: The composition does achieve, in both media, a complete dissolution (higher than 80%) after 5 hours. The percentage dissolved in system 2 is higher than that of composition in system 1 (acidic pH).

### Example 3

Capsules comprising immediate release microtablets and controlled release microtablets, comprising a superdisintegrant agent and a coating formed by a mixture of pH-dependent solubility and pH-independent solubility polymers.

**Table 9 - Content per Capsule**

| Ingredient | Function | mg/dose | % |
|---|---|---|---|
| Sildenafil Citrate | Active Ingredient | 140.5 | 53.42% |
| Avicel ph200 (microcrystalline cellulose) | Diluent | 80.5 | 30.61% |
| Kolidon CL (Crospovidone) | Superdisintegrant | 29.0 | 11.03 |
| Plasdone 29-32 (Povidone K30) | Binder | 5.0 | 1.90% |
| Magnesium stearate | Lubricant | 2.5 | 0.95% |
| Eudragit L100-55 (Methacrylic acid copolymer type C) | pH-Dependent Membrane | 3.0 | 1.14% |
| Eudragit RS100 (Ammonium Methacrylate type B) | pH-Independent Membrane | 2.0 | 0.76% |
| Miglyol 812 N (Medium chain triglycerides) | Plasticizer | 0.5 | 0.19% |
| Total | | 263.0 | 100.0% |

### Composition Preparation

Microtablets were prepared according to the following steps: sildenafil citrate, microcrystalline cellulose and crospovidone were mixed in a high shear mixer for 10 minutes. The mixture was granulated on the same equipment using an aqueous solution of 10%. The granules thus obtained were dried in a fixed bed dryer at a 40 °C for 4 hours. The dried granules were sieved with a 1 mm sieve and, finally, mixed with magnesium stearate for 3 minutes. The mixture was compressed into tablets using a Riva brand equipment fitted with microtablets punches. Each punch produces 20 microtablets of 2 mm in diameter and about 1.5 mm in height.

The prepared microtablets were partitioned into two equal size portions, one half for the immediate release fraction and the other half for the controlled release fraction. For the controlled release coating, a coating solution was prepared comprising a mixture of Eudragit L100-55 and Eudragit RS100 ethanol at a solid ratio of 60:40 and plasticized with Miglyol 812N. The microtablets were coated with the coating solution using a Vector brand fluid bed fitted with a 6 inches Wurster insert under the following operating conditions: input air temperature, 40 °C; air flow rate, 100 cfm; spraying pressure, 15 psi; peristaltic pump speed, 10 rpm. The microtablets were dried in the same fixed bed equipment at 40 °C for 3 hours.

Hard gelatin capsules size 1 were filled manually with immediate release microtablets containing an equivalent amount of 50 mg base sildenafil and controlled release microtablets containing an equivalent amount of 50 mg base sildenafil, accounting for a total filling weight of 263 mg per capsule with a 140 mg sildenafil citrate content.

**Table 10 - Capsule Dissolution of Example 3**

| | System 1 | System 2 |
|---|---|---|
| | (acidic media without pH change) | (pH change) |
| 30 min | 49% | 50% |
| 3 h | 63% | 77% |
| 5 h | 90% | 100% |

Conclusion: The composition does achieve, in both media, a complete dissolution (higher than 80%) after 5 hours. The percentage dissolved in system 2 (media change) is higher than the percentage dissolved by the composition in system 1 (acidic pH).

### Example 4

Capsule preparation with different therapeutically relevant doses.

Both immediate release granules and controlled release granules from Example 1 were used to fill capsules. The capsules used were rigid gelatin coni-snap capsules provided by Capsugel.

| Dose (Base Sildenafil equivalent) | 30 mg | 50 mg | 75 mg | 100 mg | 150 mg | 200 mg | 250 mg |
|---|---|---|---|---|---|---|---|
| Immediate Release Sildenafil (mg) | 5 | 25 | 30 | 50 | 50 | 100 | 100 |
| Controlled Release Sildenafil (mg) | 25 | 25 | | 50 | 100 | 100 | 150 |
| Total per Capsule (mg) | 30 | 50 | 75 | 100 | 150 | 200 | 250 |
| Weight of Immediate Release Fraction (mg) | 10.3 | 51.3 | 61.6 | 102.6 | 102.6 | 205.2 | 205.2 |
| Weight of Controlled Release Fraction (mg) | 55.7 | 55.7 | 100.3 | 111.4 | 222.8 | 222.8 | 334.2 |
| Total Weight dosage in capsule (mg) | 66.0 | 107.0 | 161.9 | 214.0 | 325.4 | 428.0 | 539.4 |
| Capsule Size | 3 | 3 | 3 | 2 | 0 | 0el | 00 |

Conclusion: By using conventional rigid gelatin capsules it is possible to dose sildenafil in a therapeutically relevant range of 30 mg to 250 mg. Tested compositions comprise an immediate release fraction containing of from 5 to 100 mg sildenafil and a controlled release fraction of from 25 to 150 mg sildenafil.

## Claims

1. A pulsed-release sildenafil pharmaceutical composition **characterized in that** it comprises an immediate-release fraction containing 5 to 100 mg sildenafil and a controlled-release fraction containing 25 to 150 mg sildenafil, wherein said controlled-release fraction consists of coated particles containing (a) a superdisintegrant (b) a coating formed from at least one pH-dependent solubility polymer and at least one pH-independent solubility polymer, and (c) optionally, other pharmaceutical excipients.

2. A pharmaceutical composition according to claim 1, comprising sildenafil levels higher than 45% by weight, preferably levels greater than 60% by weight.

3. A pharmaceutical composition according to claim 1, wherein said coated particles are either spherical granules or microtablets.

4. A pharmaceutical composition according to claim 1, wherein said superdisintegrant agent is selected from sodium croscarmellose, sodium starch glycolate and crospovidone.

5. A pharmaceutical composition according to claim 1, wherein said pH-dependent solubility and pH-independent solubility polymers are selected from cellulose acetate phthalate, hydroxypropylmethyl cellulose phthalate, hydroxypropylmethyl cellulose acetate succinate, polyvinyl acetate phthalate and methacrylic acid copolymers.

6. A pharmaceutical composition according to claim 1, wherein pH-independent solubility polymers are selected from ammonium methacrylate copolymers, ethyl cellulose, polyvinyl acetate, and neutral ethyl acrylate and methyl acrylate-based copolymers.

7. A pharmaceutical composition according to claim 4, wherein said superdisintegrant is crospovidone.

8. A pharmaceutical composition according to claim 5, wherein said pH-dependent solubility polymer is methacrylic acid type C copolymer (Eudragit L100-55).

9. A pharmaceutical composition according to claim 6, wherein said pH-independent solubility polymer the ammonium methacrylate type B copolymer (Eudragit RS100).

10. A pharmaceutical composition according to claim 1, wherein when measuring the sildenafil dissolution of said composition in an USP apparatus 1 (baskets), at 100 rpm in a 900 ml media of 0.01 M hydrogen chloride solution for two hours and in a pH 6.0 media of 0.5% sodium chloride solution and 1% lauryl sulfate for the remaining time, the dissolved percentage is between 40 and 60% within 30 minutes, between 60 and 80% within 3 hours and not less than 80% in 5 hours.

11. A process for preparing the composition of claim 1, wherein said process comprises the steps of: 1) forming an immediate release fraction; 2) forming a controlled release fraction; and 3) filling capsules with the required amount of each fraction.

12. The process according to claim 11, wherein the step of forming the controlled release fraction is performed by depositing the active ingredient over an inert core.

13. The process according to claim 12, wherein depositing the active ingredient over an inert core consists of: a) adding ethanol, purified water or a mixture of ethanol and purified water into a reactor of suitable capacity; b) adding a binder agent and maintaining stirring until complete dissolution thereof; c) adding sildenafil and the superdisintegrant agent and maintaining stirring until achieving a homogenous dispersion; d) spraying the slurry thus obtained over inert cores using a fluid bed fitted with a Wurster insert or tangential rotor; e) drying the granules obtained in either a fixed bed or fluid bed dryer; f) preparing a coating solution or slurry consisting of least one pH-independent solubility polymer and at least one pH-dependent solubility polymer, either dissolved or dispersed in a suitable solvent; g) spraying the obtained coating solution or slurry over said granules using a Wurster or tangential rotor type fluid bed; and (h) drying said coated granules until a constant weight is achieved.

14. The process according to claim 11, wherein the step of forming the controlled release fraction is performed by microtablet compression.

15. The process according to claim 14, wherein the microtablet compression method consists of: a) mixing sildenafil with a diluent and a superdisintegrant agent in a suitable mixer; b) granulating said mixture using a binder agent solution; c) drying and sieving the obtained granulate; d) mixing with lubricant and compressing using microtablet punches; e) preparing a coating solution or slurry consisting of at least one pH-independent solubility polymer and at least one pH-dependent solubility polymer, either dissolved or dispersed in a suitable solvent; f) spraying the coating solution or slurry thus obtained over said microtablets using a Wurster or tangential rotor type fluid bed; and g) drying said coated microtablets until a constant weight is achieved.
